# EUROPEAN PATENT APPLICATION

(11) **EP 4 427 753 A1**
(43) Date of publication of application: **11.09.2024**
(21) Application number: 23177216.1
(22) Date of filing: 05.06.2023
(51) Int. Cl.: A61K 31/7068, A61K 39/00, A61K 45/06, A61P 35/00, A61P 37/00, A61P 37/04

(54) **IMMUNOMODULATOR COMPOSITION COMPRISING AZVUDINE**

(30) Priority: 10.03.2023 CN 202310232465
(71) Applicant: Henan Genuine Biotech Co., Ltd., Henan 467036 (CN)
(72) Inventor: LI, Pan, Pingdingshan, Henan, 467036, (CN); JIA, Limin, Pingdingshan, Henan, 467036 (CN); QIN, Zhiyong, Pingdingshan, Henan, 467036 (CN); WANG, Zhaoyang, Pingdingshan, Henan, 467036 (CN)
(74) Representative: Hoffmann Eitle

(57) **Abstract**

The present invention discloses the inhibitory effect of azvudine as an immunomodulator on mice bearing CT-26 tumor. It is found that azvudine has no significant anti-tumor effect on immunodeficient B-NDG mice bearing CT-26 tumor, yet has an antitumor effect on BALB/c mice with normal immunity bearing CT-26 tumor model. The present invention further studies the combined effect of azvudine and PD-1 antibody, and finds that compared with a single drug, the combined administration has a synergistic anti-tumor effect.

## Description

### FIELD

The present invention belongs to the field of biomedicine, and in particular to an immunomodulator composition comprising azvudine.

### BACKGROUND

Deoxycytidine kinase (DCK) is an enzyme with broad substrate specificity, which can phosphorylate pyrimidine and purine deoxynucleosides, and it is a key enzyme in the remedial pathway of deoxynucleotide biosynthesis. It is capable of maintaining normal DNA metabolism and phosphorylating a variety of antiviral and anticancer nucleoside analog drugs, which can only be activated after phosphorylation, thereby inhibiting tumor growth. In the past decades, apoptosis has been widely studied, and radiotherapy strategies targeting apoptosis have become one of the important means of tumor treatment.

Azvudine is a broad-spectrum RNA virus inhibitor. As a synthetic nucleoside analog of viral RNA-dependent RNA polymerase (RdRp), it is metabolized in cells into 5'-triphosphate metabolite (azvudine triphosphate) with antiviral activity, which can specifically act on the novel coronavirus polymerase (RdRp). It targets virus RdRp, and can block the synthesis and replication of RNA chain by inhibiting the activity of RdRp in the host cell. In July 2021, azvudine tablet was approved for marketing in China for the treatment of HIV-1 infected adult patients with high viral load. In July 2022, azvudine was approved for the treatment of novel coronavirus infection.

Patent document CN201010506595.X discloses use of azvudine in the treatment of tumors, such as colon cancer, liver cancer, gastric cancer, esophageal cancer, lung cancer, breast cancer, cervical cancer, leukemia, and lymphoma. It has been found that azvudine has significant inhibitory effect on various human cancer cells and transplanted tumors in animals.

On the basis of the prior art, the present invention conducted further research on azvudine, and found that in addition to being used in broad-spectrum antiviral drugs and antitumor drugs, azvudine further has good immunomodulatory effects, which can be used as an immunemodulator, and can significantly inhibit tumor progression when administered in combination with PD-1.

### SUMMARY

The present invention provides a pharmaceutical composition comprising azvudine as an immunomodulator. Specifically, the present invention provides the following technical solutions:

In one aspect, the present invention provides a pharmaceutical composition comprising azvudine as an immunomodulator.

In a preferred embodiment of the present invention, the pharmaceutical composition further comprises a second immunomodulatory active substance.

In a preferred embodiment of the present invention, the second immunomodulatory active substance is PD-1/PD-L1 antibody or a combination thereof.

In another aspect, the present invention further provides use of azvudine as an effective active substance in regulating immune function.

In another aspect, the present invention further provides use of azvudine in the manufacture of a medicament for regulating immune function.

In another aspect, the present invention further provides azvudine as an effective active substance for use in regulating immune function.

In another aspect, the present invention further provides use of a pharmaceutical composition comprising azvudine as an effective active substance in regulating immune function.

In another aspect, the present invention further provides use of a pharmaceutical composition comprising azvudine in the manufacture of a medicament for regulating immune function.

In another aspect, the present invention further provides a pharmaceutical composition comprising azvudine as an effective active substance for use in regulating immune function.

In a preferred embodiment of the present invention, the pharmaceutical composition further comprises a second immunomodulatory active substance.

In a preferred embodiment of the present invention, the second immunomodulatory active substance is PD-1/PD-L1 antibody or a combination thereof.

In a preferred embodiment of the present invention, the PD-1 antibody is selected from the group consisting of pembrolizumab, nivolumab, sintilimab, toripalimab, RMP1-14, camrelizumab, tislelizumab, cemiplimab and a combination thereof.

In a preferred embodiment of the present invention, the PD-L1 antibody is selected from the group consisting of avelumab, atezolizumab, durvalumab and a combination thereof.

In a preferred embodiment of the present invention, the PD-1 antibody is selected from a humanized antibody having the same complementarity determining region as an RMP1-14 antibody.

In another aspect, the present invention further provides a method for regulating or enhancing immune response *in vivo,* comprising administering azvudine to a subject in need thereof, especially administering an effective amount of azvudine to a subject in need thereof.

In another aspect, the present invention further provides azvudine for use in regulating or enhancing immune response *in vivo.*

In another aspect, the present invention further provides a method for enhancing immune response, comprising obtaining a sample from a subject; isolating immune cells from the sample; culturing the immune cells with azvudine; expanding the immune cells and reintroducing the cells into the subject; and enhancing the immune response.

In another aspect, the present invention further provides azvudine for use in enhancing immune response.

In another aspect, the present invention further provides a pharmaceutical composition comprising azvudine for use in enhancing immune response.

In yet another aspect, the present invention further provides a method for enhancing immune response, comprising obtaining a sample from a subject; isolating immune cells from the sample; culturing the immune cells with a pharmaceutical composition comprising azvudine; expanding the immune cells and reintroducing the cells into the subject; and enhancing the immune response.

In a preferred embodiment of the present invention, the immune cell is selected from the group consisting of an antigen-presenting cell, a T cell, a B cell and a natural killer cell.

In a preferred embodiment of the present invention, the pharmaceutical composition further comprises a second immunoregulatory active substance.

In a preferred embodiment of the present invention, the second immunoregulatory active substance is PD-1/PD-L1 antibody or a combination thereof.

In a preferred embodiment of the present invention, the PD-1 antibody is selected from the group consisting of pembrolizumab, nivolumab, sintilimab, toripalimab, RMP1-14, camrelizumab, tislelizumab, cemiplimab and a combination thereof.

In a preferred embodiment of the present invention, the PD-L1 antibody is selected from the group consisting of avelumab, atezolizumab, durvalumab and a combination thereof.

In another aspect, the present invention further provides use of azvudine in improving the infiltration and proliferation of immune cells.

In another aspect, the present invention further provides azvudine for use in improving the infiltration and proliferation of immune cells.

In a preferred embodiment of the present invention, the immune cell is selected from the group consisting of a B cell, a T cell and an NK cell.

In another aspect, the present invention further provides use of azvudine in modulating the release of cytokines in tumor tissue or a serum sample.

In another aspect, the present invention further provides azvudine for use in modulating the release of cytokines in tumor tissue or a serum sample.

In a preferred embodiment of the present invention, the cytokine is selected from the group consisting of IFNγ, IFNβ, TNFa, GM-CSF, IL10, IL2, IL4, IL8 and MCP-1.

### BRIEF DESCRIPTION OF DRAWINGS

FIG. 1 shows the flow cytometry gating.
FIG. 2 shows the ratio of CD45+/T/NK/Myeloid cells and the number of cells per 100 mg of tumor.
FIG. 3 shows the ratio of CD4+T/CD8+T/Treg and the number of cells per 100 mg of tumor.
FIG. 4 shows the ratio of DC/M-MDSC/PMN-MDSC and the number of cells per 100 mg of tumor.
FIG. 5 shows the ratio of CD8+T to Treg and the ratio of CD4+Teff to Treg.
FIG. 6 shows the CD69 positive rate and median fluorescence intensity in T, CD4+T, and CD8+T cells.
FIG. 7 shows the Ki67 positive rate and median fluorescence intensity in T, CD4+T, and CD8+T cells.
FIG. 8 shows the expression amount of GM-CSF, MCP-1, IFNγ, IFNβ, TNFa, IL2, IL4, IL8, and IL10.
FIG. 9 shows the effect of azvudine in combination with PD-1 antibody on tumor volume in murine colorectal cancer CT-26 homograft tumor model.
FIG. 10 shows the effect of azvudine in combination with PD-1 antibody on tumor volume in B-cell lymphoma A20 tumor-bearing mouse model

### DETAILED DESCRIPTION

Example 1 Comparison of the anti-tumor effect of azvudine on the mice with normal immune function and mice with severe immunodeficiency.

### Experimental Materials

### Experimental animals and feeding environment

### Experimental animals

Species: Mouse
Strain: BALB/c mouse
Age and weight: 6-8 weeks old, 18.7-22.5 g
Gender: Female
Supplier: Shanghai Lingchang Biotechnology Co., Ltd.

### Information on the test product and control product

Name: Azvudine; Characteristic description: Powder; Purity: 100%; Molecular weight: 286.08; Molecular weight with salt: 286.22; Packaging: 30.5 mg/bottle; Storage condition: 4°C.
Name: Anti-mouse PD-1 (CD279); Provider: Bioxcell; Characteristic description: Liquid; Packaging: 10.29 mg/ml.
Storage condition: 4°C.

### Cell culture

CT26 cells were cultured in RPMI 1640 medium containing 10% fetal bovine serum at 37°C in a 5% CO₂ incubator. The cells were passaged routinely once a week. When the cell saturation reached 80%-90% and the cell number achieved the requirement, the cells were collected, counted and inoculated.

### Tumor cell inoculation

0.1 mL (0.3×10⁶) of CT26 cells were subcutaneously inoculated into the right lower limb of each mouse. When the average tumor volume reached about 51 mm³, the mice were grouped and administered.

Experimental process: BALB/c mice with normal immunity were selected, which had a genetic background of inbred strain, genetically bred by brothers and sisters, and thus had little individual differences, purer genetic genes, and better overall quality. B-NDG mice were mice with severe immunodeficiency independently developed by Biocytogen, wherein the IL2rg gene on the NOD-scid mouse background was knocked out, and the mice showed a lack of mature T, B, and NK cells.

The first group was the blank group, the second group was the mice with normal immunity administered with 0.25mpk, the third group was the mice with normal immunity administered with 1mpk, the fourth group was the mice with severe immunodeficiency administered with 0.25mpk, the fifth group was the mice with severe immunodeficiency administered with 1mpk. The administration route was o.p., and the administration cycle was QD×2W.

The experimental index was to investigate whether tumor growth was inhibited, delayed or cured. Tumor diameter was measured with a vernier caliper three times a week. The tumor volume was calculated according to the formula of: V= 0.5*a* × *b*², where *a* and *b* represented the long diameter and short diameter of the tumor, respectively.

The anti-tumor efficacy of the compounds was evaluated by TGI (%) or relative tumor proliferation rate T/C (%). TGI (%) reflected the inhibition rate of tumor growth. TGI (%) was calculated according to the formula of: TGI (%)= [1-(average tumor volume at the end of administration of a certain treatment group - average tumor volume at the beginning of administration of this treatment group)/(average tumor volume at the end of treatment of solvent control group - average tumor volume at the beginning of treatment of the solvent control group)]×100%.

Relative tumor proliferation rate T/C (%) was calculated according to the formula as follows: T/C % = T_{RTV} / C_{RTV} × 100 % (T_{RTV} represented the average RTV of the treatment group; C_{RTV} represented the average RTV of the negative control group). Relative tumor volume (RTV) was calculated based on the results of measurement of tumor according to the formula of RTV = Vₜ/V₀, where V₀ represented the tumor volume measured at the time of grouping and administering (i.e. d₀), and Vₜ represented the tumor volume obtained in a certain measurement, and T_{RTV} and C_{RTV} were collected from the data on the same day.

### Comparison of test results

**Table 1**

| Group | TGI(%) | IR(%) | P value |
|---|---|---|---|
| The first group | - | - | - |
| The second group | 63 | 57.5 | <0.0001 |
| The third group | 94 | 84.5 | <0. 0003 |
| The fourth group | 13 | 9.4 | 0.3364 |
| The fifth group | 53 | 49.2 | <0.0001 |

It can be seen from the above test results that azvudine at both doses of 0.25 mg/kg and 1 mg/kg did not show a significant tumor inhibitory effect in immunodeficient B-NDG mice bearing CT-26 tumor, with TGIs of 13% and 53% respectively, which was lower than the anti-tumor effect of azvudine at the same doses on the BALB/c mice with normal immune function bearing CT-26 tumor model, with TGIs of 63% and 94%, respectively. It further indicated that azvudine can be used as an immunomodulator.

### Example 2 Study on effect of azvudine on infiltration of immune cells in tumor

### Preparation of single cell suspension

Tumor tissue was washed once with PBS, and removed of fat. The tumor tissue was quickly cut into small pieces. The chopped tumor tissue was transferred to a tube C containing 5 mL of digestive enzyme mixed solution. The tube C was put in a tissue processor, the tissue fragments were ensured to be submerged in the digestive enzyme, and the program m_imptumor_01_01 was performed. The sample was put in a water bath shaker at 37°C for 30 min of digestion. After the digestion was completed, the program m_imptumor_01_01 was performed again, and then an appropriate amount of PBS containing 2% fetal bovine serum was added to terminate the digestion. The obtained mixture was filtered with a 70 µm cell mesh to remove residual tissue, and washed with PBS. An appropriate volume of PBS was added according to the amount of cell precipitate to resuspend cells. Then the cells were gently blown evenly, and counted with a hemocytometer. An appropriate amount of cells were taken out and stained with antibodies.

### Extracellular staining

The cells were resuspended with PBS to a concentration of 1×10⁶ cells/100 µL. The cell suspension was added to a 96-well V-bottom plate at 100 µL per well. The plate was centrifuged at 450 g at 4°C for 5 min, and the supernatant solution was discarded. The cells were resuspended with 100 µL of prepared Zombie NIR Live/Dead solution, then cultured at 4°C in the dark for 30 min, and then washed twice with PBS. Then the cells in each well were resuspended with 50 µL of staining buffer containing Fc receptor inhibitor (1 µL of Fc receptor inhibitor + 49 µL of staining buffer), and cultured at 4°C in the dark for 5 min. Then the cells in each well were resuspended with 50 µL of Brilliant staining buffer containing Pacific Orange CD8, BV605 MHCII, BV650 CD25, BV750 CD45, BV785 Ly6C, AF488 CD11c, AF532 CD11b, PE-DZL594 CD49b, APC Ly6G, APC-Cy5.5 CD4, APC-Cy7 CD13, BV42 CD69 (or an isotype control antibody thereof), and cultured at 4°C in the dark for 30 min. After staining was completed, the cells were washed twice with 200 µL of staining buffer.

### Intracellular staining

The cells in each well were resuspended with 100 µL of Fixation/Permeabilization, and cultured at 4°C in the dark for 30 min. After permeabilization was completed, the cells were washed twice with 200 µL of Permeabilization buffer. The cells were resuspended with 100 µL of Permeabilization buffer containing Fc receptor inhibitor, and cultured at 4°C in the dark for 5 min. Then the cells were directly added with intracellular antibodies PE-Cy7 FoxP3 and PE Ki67 (or an isotype control antibody thereof), and cultured at 4°C in the dark for 30 min. After staining was completed, the cells were washed twice with 200 µL of Permeabilization buffer, and resuspended with 200 µL of staining buffer. The flow cytometry detection was completed within 24 h.

### Data analysis

The flow cytometry data was analyzed using FlowJo, SPSS and Graphpad Prism softwares, specifically including the following:
1) The proportion of CD45+ cells in living cells of tumor tissue;
2) The ratio of T, CD4+ T, CD8+ T, Treg, Myeloid, DC, NK, PMN-MDSC and M-MDSC in CD45+ cells;
3) The ratio of CD8+ T to Treg and the ratio of CD4+ Teff to Treg;
4) The CD69 positive rate and median fluorescence intensity (MFI) in T, CD4+ T, and CD8+ T cells;
5) The Ki67 positive rate and median fluorescence intensity (MFI) in T, CD4+ T, CD8+ and T cells;
6) The number of T, CD4+ T, CD8+ T, Treg, Myeloid, DC, NK, PMN-MDSC, M-MDSC, and white blood cells per 100 mg of tumor tissue;

### Method of administration

**Table 2**

| Compound treatment | | Dose (mg/kg) | | Administrati on route | Administration regimen |
|---|---|---|---|---|---|
| | Vehicle | | - | PO | QD×5D |
| | Azvudine | | 0.25 | PO | QD×5D |
| | Azvudine | | 1 | PO | QD×5D |

In this experiment, the CT26 tumor model was established on BALB/c mice, and the mice were treated with azvudine at different doses (0.25 mg/kg, 1 mg/kg). Tumor samples were collected. Then the proportion of each immune cell subset and the activation and proliferation of T, CD4+ T and CD8+ T were detected by flow cytometry.

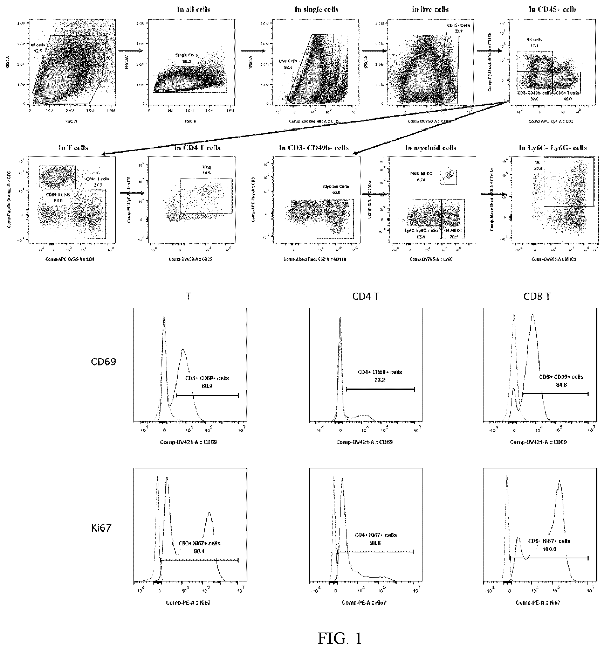

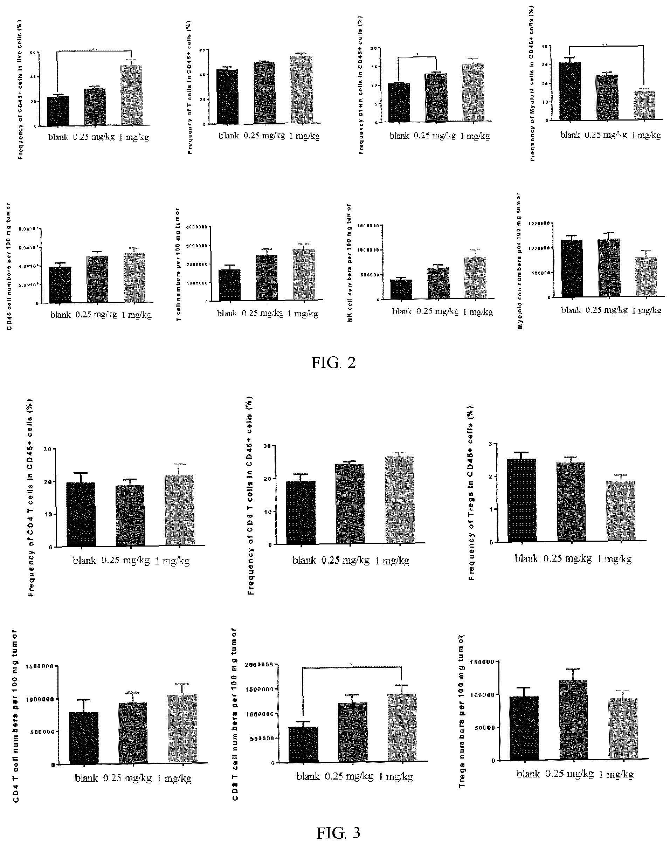

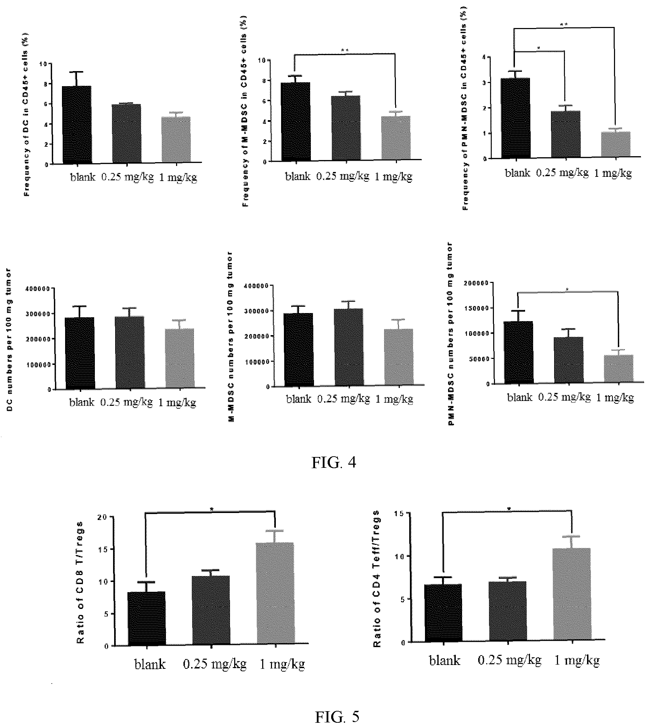

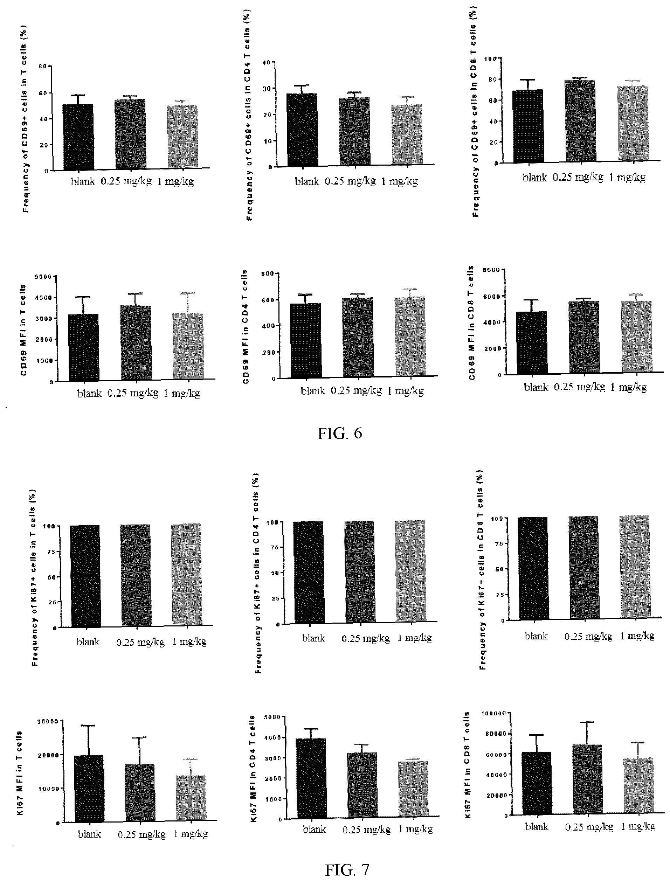

After administration, the infiltration of immune cells in the tumor increased, mainly due to the increase in infiltration of CD8+ T and NK cells, which was dose-dependent. The ratios of CD8T/Treg and CD4Teff/Treg in the high-dose group were significantly increased; and the proportion of myeloid cells in the high-dose group significantly reduced, mainly due to the significant reduction in the proportion of M-MDSC and PMN-MDSC cells. The low-dose group also had the same trend and showed a certain dose-dependence. Other cell subsets after administration showed no significant change compared with the control group, and the expression levels of Ki67 and CD69 in T, CD4+ T, and CD8+ T cells also showed no significant difference (the experimental results are shown in FIG. 1-FIG. 7, wherein in FIG. 2-FIG. 7, "blank" represents the blank group, "0.25 mg/kg" represents the administration group with administration at 0.25 mg/kg, and "1 mg/kg" represents the administration group with administration at 1 mg/kg).

Based on this experiment, azvudine can play an anti-tumor effect by inhibiting the infiltration and proliferation of M-MDSC and PMN-MDSC in tumors and promoting the infiltration and proliferation of CD8+ T/NK cells, further indicating that azvudine can act as an immunomodulator.

### Example 3 Effect of azvudine on the release level of cytokines in serum and tumor tissue

### Cell culture:

CT26 cells were cultured in RPMI 1640 medium containing 10% fetal bovine serum at 37°C in a 5% CO₂ incubator. The cells were passaged routinely once a week. When the cell saturation reached 80%-90% and the cell number achieved the requirement, the cells were collected, counted and inoculated.

### Animal:

Animals were BALB/c female mice, 8 weeks old and weighing 18.5-22.9 g, which were provided by Shanghai Lingchang Biotechnology Co., Ltd.

### Tumor inoculation:

0.1 mL (0.3×10⁶) of CT26 cells were subcutaneously inoculated into each mouse. When the average tumor volume reached 101 mm³, the mice were grouped and administered.

### Administration method:

The *in vivo* administration method of this experiment is shown in the table below.

**Table 3. In vivo administration method**

| Compound treatment | | Dose (mg/kg) | Administrati on route | Administration regimen |
|---|---|---|---|---|
| | Vehicle | - | PO | QD×5D |
| | Azvudine | 0.25 | PO | QD×5D |
| | Azvudine | 1 | PO | QD×5D |

### Protein extraction and quantification

1) Quick-frozen tissue samples were taken out from the -80°C refrigerator.
2) On dry ice, part of the tumor tissue (about 50-100 mg) were cut out, and put into a 2 mL centrifuge tube with steel beads. Then the tube was added with 350 µL of cell lysis solution RIPA (in which 1% protease inhibitor and phosphatase inhibitor had been freshly added).
3) The tissue was crushed with Tissue grinder at the highest frequency for 5 min.
4) The tissue lysate was put on ice for 30 min.
5) The tissue lysate was centrifuged at 12,000 rpm at 4°C for 10 min. The supernatant was collected and put into a new 1.5 mL centrifuge tube.

### Tumor tissue protein quantification method

1) The protein extract was diluted 50 times (2 µL of protein extract + 98 µL of RIPA cell lysis buffer).
2) Standard product and diluted samples were added at 10 µL/well in a 96-well transparent plate.
3) Preparation of working solution: Reagent A and reagent B were mixed at a ratio of 50:1. 200 µL of the mixture was added to each well.
4) The 96-well transparent plate was incubated at 37°C for 30 min.
5) The OD value at 562 nm was detected by a microplate reader, and then the tumor protein was quantified according to the OD result.
6) The total protein concentration of the tumor samples was uniformly 2 mg/mL.
7) The protein was quantified with BCA quantification kit.
8) According to the quantitation results, the sample protein concentration was uniformly diluted to 2 µg/µL with RIPA lysis solution.
9) MSD experiment was performed or the diluted samples were stored in a -80°C refrigerator.

### MSD cytokine detection

### Preparation of MSD reagent

1) Preparation of standard product: 250 µL of Diluent 43 diluent was added to the standard product. The mixture was mixed well, and left to stand at room temperature for 15-30 min, and serially diluted by 4 times using Diluent 43 to obtain standard products Nos. 1-7. Diluent 43 was standard product No. 8.
2) Sample dilution: The samples were diluted with Diluent 43.
3) Antibody dilution: 100X antibody stock solution was diluted to 1X.
4) Buffer preparation: 5X buffer was diluted to 1X with deionized water.

### MSD detection

1) 200 µL of each biotin antibody was added to 300 µL of the designated linker. The resulting mixture was stirred by vortex and incubated at room temperature for 30 min.
2) 200 µL of stop solution was added. The resulting mixture was stirred by vortex and incubated at room temperature for 30 min.
3) 600 µL of each U-PLEX Linker-Coupled antibody solution (10X) was mixed into a tube and mixed by vortex.
4) When the number of binding antibodies was less than 10, the solution was supplemented to 6 ml with stop solution.
5) 50 µL of multiplex coating solution was added to each well. The plate was sealed with an adhesive board, and shaken at room temperature for 1 h.
6) The wells were washed 3 times with 1X MSD wash Buffer at 150 µL/well.
7) Standard product and detection antibody solutions were prepared.
8) 25 µL of Diluent 43 was added to each well.
9) 25 µL of prepared standard product, tissue sample or serum sample was added to each well. The plate was sealed with an adhesive board, and incubated at room temperature for 1 h.
10) The wells were washed 3 times with 1X MSD wash Buffer at150 µL/well.
11) 50 µL of detection antibody solution was added to each well. The plate was sealed with an adhesive board, and incubated at room temperature for 1 h.
12) The wells were washed 3 times with 1X MSD wash Buffer at150 µL/well.
13) 150 µL of LMSD GLOD Read Buffer was added to each well. The plate was detected on an MSD instrument.

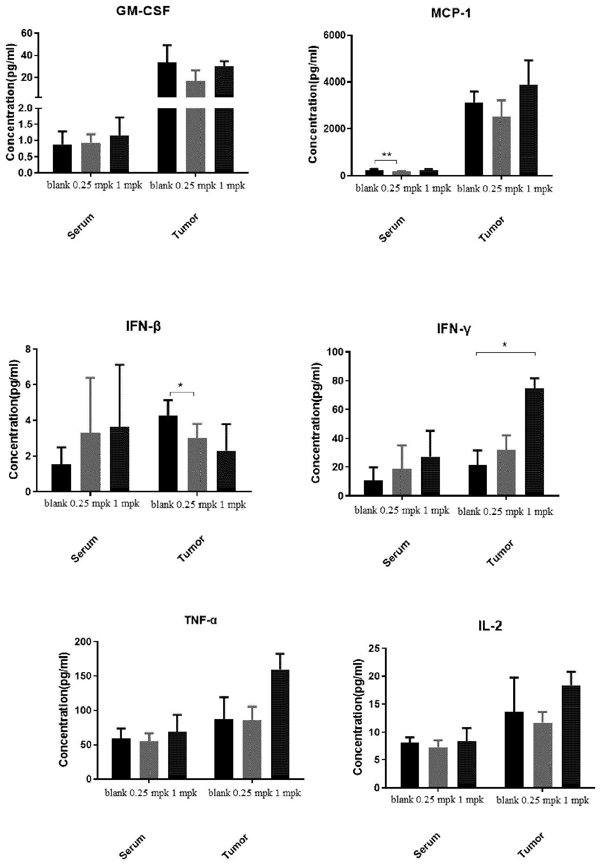

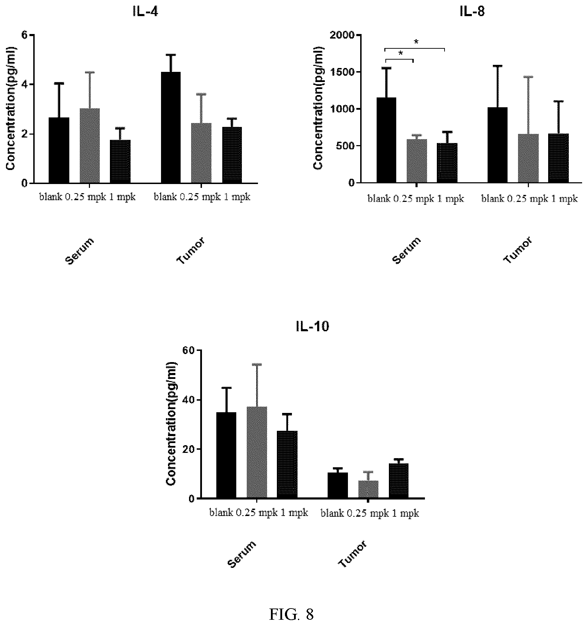

In this experiment, a CT26 tumor model was established on BALB/c mice. The mice were treated with azvudine at different doses (0.25 mg/kg, 1 mg/kg) for 5 days. The serum and tumor samples were collected 2 h after the last administration. The release levels of cytokines (GM-CSF, MCP-1, IFNγ, IFNβ, TNFa, IL2, IL4, IL8, IL10) in serum and tumor tissue were detected by MSD method (FIG. 8). In FIG. 8, "blank" represents the blank group, "0.25 mpk" represents the administration group with administration at 0.25 mg/kg, and "1 mpk" represents the administration group with administration at 1 mg/kg.

In serum samples, compared with the Vehicle group, the release of MCP-1 in the azvudine 0.25 mg/kg treatment group was significantly reduced, the release of IL8 in the azvudine 0.25 mg/kg and 1 mg/kg treatment groups was significantly reduced, and other cytokines showed no significant change. In tumor tissue, compared with the Vehicle group, in the azvudine 0.25 mg/kg and 1 mg/kg treatment groups, the release of IFNγ and TNFα increased, and the release of IFN-β decreased, which showed certain dose-dependency. The above experimental results show that azvudine has a certain regulatory effect on the release of cytokines.

### Example 4 Study of the combination of azvudine and PD-1 antibody in murine colorectal cancer CT-26 homograft tumor model

Further, the anti-tumor effect of azvudine in combination with PD-1 antibody on BALB/c female mouse subcutaneously xenografted with murine colorectal cancer CT-26 cell line animal model was studied.

**Table 4**

| Group | Compound treatment | Dose (mg/kg) | Administration volume parameter (µL/g)² | Vehicle | Administrati on route | Administration frequency |
|---|---|---|---|---|---|---|
| 1 | Blank | - | 10 | Normal saline | p.o. | QD× 18 days |
| 2 | Azvudine | 0.25 | 10 | Normal saline | p.o. | QD× 18 days |
| 3 | Azvudine | 0.5 | 10 | Normal saline | p.o. | QD× 18 days |
| 4 | anti-PD-1 | 10 | 10 | PBS | i.p. | BIW × 3 weeks |
| 5 | Azvudine 1 | 0.5 | 10 | Normal saline+PB S | p.o.+i.p. | QD×18 days + BIW × 3 weeks |
| | anti-PD-1 | 10 | | | | |

The test results are analyzed as shown in the following table:

**Table 5 Evaluation of anti-tumor effect of the test drugs on the CT26 homograft tumor model (calculated based on the tumor volume on the 17^{th} day after administration)**

| Treatment | Tumor volume (mm³)^{a} on the 17^{th} day | T/C (%) | TGI^{b} (%) | p value (%) |
|---|---|---|---|---|
| Vehicle | 2004±255 | -- | -- | -- |
| Azvudine (0.25mg/kg) | 768±74 | 39.18 | 63.26 | <0.0001 |
| Azvudine (0.5mg/kg) | 347±77 | 17.63 | 84.85 | <0.0001 |
| anti-PD-1 (10mg/kg) | 555±111 | 29.25 | 74.16 | <0.0001 |
| anti-PD-1+Azvudine (10+0.5mg/kg) | 119±48 | 5.66 | 96.5 | <0.0001 |

**Table 6. Tumor weight in each group**

| Group | Tumor weight (mg) (On the 17^{th} day) | T/C_{weight} (%) | p value |
|---|---|---|---|
| Vehicle | 1994±863 | -- | -- |
| Azvudine (0.25mg/kg) | 848±210 | 42.52 | <0.0001 |
| Azvudine (0.5mg/kg) | 386±256 | 19.37 | <0.0001 |
| anti-PD-1 (10mg/kg) | 577±308 | 28.92 | <0.0001 |
| anti-PD-1+Azvudine (10+0.5mg/kg) | 127±163 | 6.39 | <0.0001 |

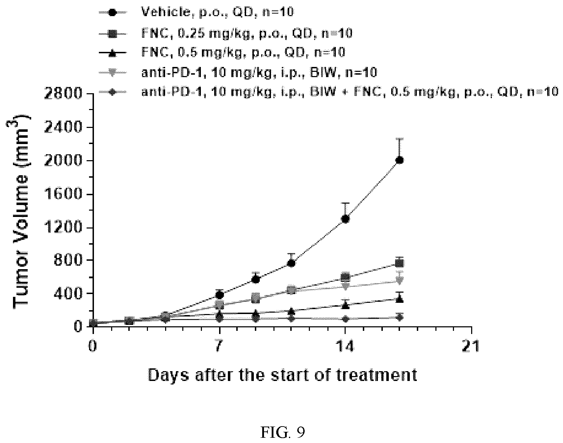

In this experiment, the *in vivo* efficacy of the test drug on the CT26 homograft tumor model was evaluated. The tumor volumes of each group at different time points are shown in Table 5, Table 6 and FIG. 9. On the 17^{th} day after administration, the tumor volume of the tumor-bearing mice in the blank control group reached 2,004 mm³. Compared with the blank control group, azvudine (0.25 mg/kg) group showed a significant tumor inhibitory effect, with a tumor volume of 768 mm³, T/C of 39.18%, TGI of 63.26%, and p-value of <0.0001. Compared with the blank control group, azvudine (0.5 mg/kg) group showed a significant tumor inhibitory effect, with a tumor volume of 347 mm³, T/C of 17.63%, TGI of 84.85%, and p value of <0.0001. Compared with the blank control group, the test drug combination group of azvudine+anti-PD-1 (0.5+10 mg/kg) showed a significant tumor inhibitory effect, with a tumor volume of 119 mm³, T/C of 5.66%, TGI of 96.5%, and p-value of <0.0001. The analysis and statistical results of the tumor weight in the test drug combination group were basically consistent with the tumor volume data.

The body weight of experimental animals was used as a reference index for indirect determination of drug toxicity. The tumor-bearing mice showed good tolerance to the test drugs with different doses, and none of the treatment groups showed significant weight loss.

In this experiment, the test drugs azvudine (0.25 mg/kg), azvudine (0.5 mg/kg) and azvudine+anti-PD-1 (0.5+10 mg/kg) under test doses had significant inhibitory effect on CT26 homograft tumor growth. In this experiment, the test drugs azvudine (0.5 mg/kg) in combination with anti-PD-1 can improve the tumor inhibitory effect of single drug azvudine and single drug anti-PD-1 in this CT26 model, and TGI was increased from 84.85% and 74.16% to 96.5% respectively.

### Example 5 Study of the combination of azvudine and PD-1 antibody in B-cell lymphoma A20 tumor-bearing mouse model

The effect of azvudine in combination with PD-1 antibody on the B-cell lymphoma A20 tumor-bearing mouse model was further studied. Both PD-1 and azvudine had certain inhibitory activity on the B-cell lymphoma A20 tumor-bearing mice when administered alone. When azvudine was administered in combination with PD-1 antibody, the tumor progression in mice was significantly inhibited.

**Table 7**

| Group | TGI(%) | IR(%) | P value |
|---|---|---|---|
| Blank group | - | - | - |
| Azvudine single drug group (1mpk) | 70 | 70.8 | <0.0001 |
| PD-1 antibody single drug group (10mpk) | 35 | 34.6 | <0.0006 |
| Azvudine group+PD-1 antibody (1 mpk+1 0mpk) | 81 | 80.1 | <0.0001 |

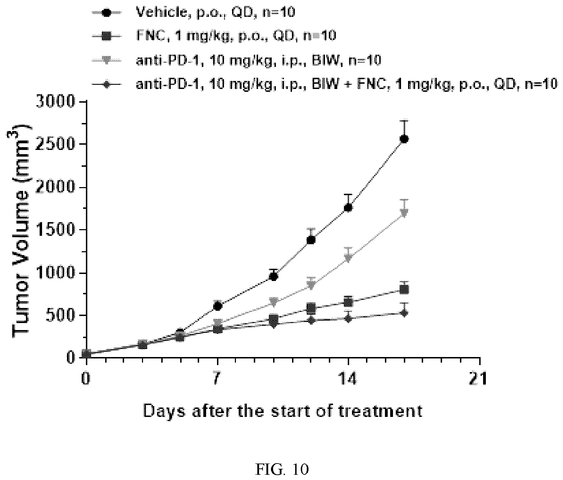

As shown in Table 7 and FIG. 10, azvudine at both doses of 1 mg/kg and 2 mg/kg, and azvudine in combination with anti-PD-1 (1+10 mg/kg) showed significant inhibitory effect on A20 homograft tumor growth, with TGIs of 70%, 88% and 81% respectively. Azvudine 1 mg/kg in combination with anti-PD-1 can improve the anti-tumor effect of single drug anti-PD-1 in this A20 B-cell lymphoma, and TGI was increased from 34.8% to 81%.

Although the specific embodiments of the present invention have been described above, those skilled in the art should understand that these are only examples, and various changes or modifications can be made to these embodiments without departing from the principle and essence of the present invention. Accordingly, the protection scope of the present invention is defined by the claims.

## Claims

1. A pharmaceutical composition comprising azvudine as an immunomodulator.

2. The pharmaceutical composition according to claim 1, further comprising a second immunomodulatory active substance;
wherein the second immunomodulatory active substance is PD-1/PD-L1 antibody or a combination thereof.

3. A pharmaceutical composition comprising azvudine as an effective active substance for use in regulating immune function.

4. The pharmaceutical composition for use according to claim 3, wherein the pharmaceutical composition further comprises a second immunoregulatory active substance.

5. The pharmaceutical composition for use according to claim 4, wherein the second immunomodulatory active substance is PD-1/PD-L1 antibody or a combination thereof.

6. The pharmaceutical composition for use according to claim 5, wherein the PD-1 antibody is selected from the group consisting of pembrolizumab, nivolumab, sintilimab, toripalimab, RMP1-14, camrelizumab, tislelizumab, cemiplimab and a combination thereof; or
the PD-L1 antibody is selected from the group consisting of avelumab, atezolizumab, durvalumab and a combination thereof; or
the PD-1 antibody is selected from a humanized antibody having the same complementarity determining region as an RMP1-14 antibody.

7. Azvudine for use in regulating or enhancing immune response *in vivo.*

8. A pharmaceutical composition comprising azvudine for use in enhancing immune response.

9. The pharmaceutical composition for use according to claim 8, wherein the pharmaceutical composition further comprises a second immunoregulatory active substance.

10. The pharmaceutical composition for use according to claim 9, wherein the second immunoregulatory active substance is PD-1/PD-L1 antibody or a combination thereof.

11. The pharmaceutical composition for use according to claim 10, wherein the PD-1 antibody is selected from the group consisting of pembrolizumab, nivolumab, sintilimab, toripalimab, RMP1-14, camrelizumab, tislelizumab, cemiplimab and a combination thereof; or
the PD-L1 antibody is selected from the group consisting of avelumab, atezolizumab, durvalumab and a combination thereof.

12. Azvudine for use in improving the infiltration and proliferation of immune cells.

13. The azvudine for use according to claim 12, wherein the immune cell is selected from the group consisting of a B cell, a T cell and an NK cell.

14. Azvudine for use in modulating the release of cytokines in tumor tissue or a serum sample.

15. The azvudine for use according to claim 14, wherein the cytokine is selected from the group consisting of IFNγ, IFNβ, TNFa, GM-CSF, IL10, IL2, IL4, IL8 and MCP-1.
